# EUROPÄISCHE PATENTANMELDUNG

(11) **EP 1 396 497 A1**
(43) Veröffentlichungstag der Anmeldung: **10.03.2004**
(21) Anmeldenummer: 03019943.4
(22) Anmeldetag: 02.09.2003
(51) Int. Cl.: C07H 19/067, C07H 1/08, A61K 7/00, A23L 1/30, A23K 1/02

(54) **Verfahren zur Gewinnung von Uridin aus Melasse**

(30) Priorität: 03.09.2002 DE 10241116
(71) Anmelder: AMINO GmbH, D-38373 Frellstedt (DE)
(72) Erfinder: Busse, Matthias, 38162 Schulenrode (DE); Faurie, Robert Dr. rer. nat., 38154 Königslutter (DE); Danneel, Hans-Jürgen Prof. Dr. rer. nat., 32689 Kalletal (DE)
(74) Vertreter: Einsel, Martin

(57) **Zusammenfassung**

Die vorliegende Erfindung betrifft ein Verfahren zur Gewinnung von Uridin aus Melasse mittels chromatographischer Verfahren, wobei Uridin angereichert und in hoher Ausbeute und hoher Reinheit erhalten und insbesondere Uracil von Uridin abgetrennt wird.

## Beschreibung

Die vorliegende Erfindung betrifft ein Verfahren zur Gewinnung von Uridin aus Melasse auf Basis chromatographischer Methoden.

Melasse fällt bei der Zuckergewinnung aus Zuckerrüben aufgrund des hohen Trockensubstanzanteils als hochviskoser klebriger Sirup an. Dieser Trockensubstanzanteil setzt sich neben Saccharose als Hauptbestandteil aus einer großen Menge wertvoller Pflanzeninhaltsstoffe zusammen.

Beispiele hierfür sind weitere Saccharide wie Oligosaccharide und nicht-Zuckerbestandteile wie Vitamine und organische Stickstoffverbindungen wie Aminosäuren und Nukleotide, beziehungsweise deren Bausteine, die Nukleoside und Nukleobasen.

Viele dieser weiteren Inhaltsstoffe sind in der Melasse jedoch nur zu einem geringen Teil von unter 1 % enthalten als sogenannte "minor components". Hierzu gehören auch die Nukleoside und Nukleobasen. So gibt Reinefeld E. et al. für Uridin in der Melasse einen Gehalt von 630 mg/dm³ beziehungsweise 882 mg/kg an (E. Reinefeld et al. "Zum Nachweis und Vorkommen von Nukleotid-Spaltprodukten in technischen Zuckerlösungen - Beitrag zur Vervollständigung der erfassten Stickstoffverbindungen" in Zuckerind. 111 (1986) Nr. 11, Seiten 1017 bis 1024).

Bei diesen Inhaltsstoffen der Melasse handelt es sich um Roh- und Wirksubstanzen, die insbesondere für pharmazeutische Zwecke und auch für die menschliche Ernährung von Interesse sind. Beispielsweise wurde der therapeutische Nutzen des Nukleinsäurebausteins Uridin in klinischen Studien und Tierexperimenten bei verschiedenen Krankheiten wie Herzgefäßerkrankung und Hypertonie, Erkrankung der Atemwege, Leberfehlfunktion, Unfruchtbarkeit, Krebs, Aids, Epilepsie, Parkinson-Krankheit, Angstneurose, Schlafstörungen, Ischemie und Hypoxie nachgewiesen. Die Fähigkeit von Uridin und einiger seiner Derivate, in das intrazelluläre Milieu einer Zelle zu gelangen, eröffnet auch für die Zukunft ein weites Einsatzgebiet für die pharmazeutische Anwendung (G. P. Connolli at al. "Uridine and its Nucleosides: Biological Actions, Therapeutic Potentials" in Trends in Pharmacol. Sci. 20 (1969), Seiten 218 bis 225 sowie zum Beispiel WO 99/08686, EP 0 348 360 und KR 9408033). Daneben ist Uridin als Nahrungsmitteladditiv insbesondere auch für die Bereiche Baby- und Sportlernahrung, von Interesse.

Aufgrund des enormen Anwendungspotentials besteht daher eine steigende Nachfrage an Uridin. Bekannte Verfahren zur Gewinnung von Ribonukleosiden wie Uridin sind die Hydrolyse von RNA, die chemisch wie in DE 824206, meist aber enzymatisch unter Nutzung von 5'-Phosphodiesterase (US 3,304,238) erfolgt. Die Gewinnung von Nukleosiden und Nukleotiden durch Hydrolyse beinhaltet die Bereitstellung eines ribonukleinsäurehaltigen Rohstoffes, die Gewinnung der RNA aus diesem, die Hydrolyse der RNA und die Trennung der aus der Hydrolyse resultierenden Bausteine. Da das Enzym 5'-Phosphodiesterase unselektiv Aktivität sowohl gegenüber RNA als auch DNA aufweist, müssen für die Gewinnung reiner RNA-Bestandteile mindestens acht verwandte Substanzen mit hohem Aufwand voneinander getrennt werden.

Weiter wurden für die Gewinnung von Uridin Fermentationsverfahren (US 4,880,736 und JP 4252190) sowie ein Biotransformationsverfahren (JP 1074998) entwickelt. Dem gegenüber der RNA-Hydrolyse verringerten Aufwand bei der Stoffreinigung steht dabei allerdings der höhere Aufwand für die gezielte Uridin-Synthese entgegen.

Melasse als mögliche Quelle für die in ihr enthaltenen Roh- und Wirkstoffe rückte in den Blickpunkt des Interesses, seitdem große Mengen Melasse durch Abtrennung des hohen Zuckeranteils als Flüssigzucker für die Lebensmittelindustrie bearbeitet werden. Die Abtrennung der Saccharosefraktion von den übrigen Melassebestandteilen erfolgt hierbei üblicherweise durch lonenausschlusschromatographie an stark sauren Kationenaustauschern (US 3,884,714, US 4,412,866, US 5,795,398). Bei diesen Verfahren fallen Nebenproduktfraktionen an, in denen sich die Melasseinhaltsstoffe abhängig von den auf sie ausgeübten Kräften anreichern können. Jedoch enthalten die Fraktionen, die bei der chromatographischen Melasseentzuckerung erhalten werden, in der Regel eine Mischung einzelner Bestandteile der Melasse, die zur Isolierung einer gewünschten Zielsubstanz weiter aufgereinigt werden müssen. Die Zusammensetzung der erhaltenen Fraktionen kann hierbei je nach Trennverhalten der einzelnen Trennsäulen sowie aufgrund der starken Schwankungen in der Melassezusammensetzung selbst erheblich variieren.

Oikawa Sh. et al. beschreibt in "Chromatographic Separation of Molasses constituents (Part 7)", Research Society of Japan Sugar Refineries Technologists, Band 31, 1982, Seiten 55 bis 63 die Gewinnung von Adenosin, einem Nukleosid, aus Dünnsaft, einem Zwischenprodukt der Zuckergewinnung. Aus dem Konzentrat von Dünnsaft wird durch Abtrennung der Saccharose mittels Kristallisation Zucker gewonnen, wobei als Nebenprodukt Melasse anfällt.
Gemäß Oikawa wird bei der Behandlung von Dünnsaft an einer an sich bekannten Kombination aus Kationenaustauscher und Anionenaustauscher zur Entsalzung Uridin im basischen Eluat des Regenerierungsmittels vom Anionenaustauscher gefunden. Eine chromatographische Abtrennung des Uridins, insbesondere von Uracil, findet jedoch nicht statt.

Aufgrund ihres sehr ähnlichen Verhaltens ist die Isolierung der einzelnen Nukleinsäurebausteine wie der Nukleotide, Nukleoside und Nukleobasen, aus den erhaltenen Fraktionen problematisch. Insbesondere hat sich dabei die Abtrennung der Nukleoside von den Nukleobasen als schwierig erwiesen.

Untersuchungen zur Auftrennung der Nukleinsäurebausteine finden sich bei Reinefeld (a. o. O.) und J.B. Stark at al. "The Purines, Pyrimidines and Nucleosides in Beet Diffusion Juice and Molasses", in J. Amer. Soc. Suger Beet Technol. 9, Seiten 201-206. Reinefeld schlägt vor, zur Abtrennung der Nukleotide von den Nukleosiden und Nukleobasen die höhere Säurestärke der Nukleotide auszunutzen. Dazu werden zunächst alle drei Bestandteile an einen stark basischen Anionenaustauscher gebunden und die Nukleoside und Nukleobasen von den Nukleotiden mittels 2n Essigsäure abgetrennt. Damit lassen sich zwar die Nukleobasen und Nukleoside von dem Austauscherharz eluieren, nicht jedoch die Nukleotide, die am Harz gebunden bleiben. Zur weiteren Auftrennung wird das Eluat mit 2n Essigsäure, das die Nukleobasen und Nukleoside enthält, auf einen stark sauren Kationenaustauscher (H⁺-Form) gegeben. Hierbei erfolgt jedoch keine Auftrennung in Nukleoside und Nukleobasen, sondern lediglich eine Auftrennung entsprechend den Basen in Purinbasen und Nukleoside mit Purinbasen sowie Cytidin und Cytosin sowie in Pyrimidinbasen und Nukleoside mit Pyrimidinbasen.
Eine Auftrennung der Nukleoside und Nukleobasen, insbesondere von Uridin und Uracil, findet nicht statt.

Stark schlägt eine Vortrennung der in der Melasse enthaltenden Purine, Pyrimidine und Nukleoside an einem Kationenaustauscher (DOWEX-50 in H⁺-Form) und anschließend eine weitere Auftrennung der erhaltenen Fraktionen an Anionenaustauschern vor. Zur Elution des Kationaustauschers wird Wasser beziehungsweise 0,4 n Ammoniumhydroxid verwendet, wobei Uridin zusammen mit Uracil und weiteren Bestandteilen in der Wasserfraktion enthalten ist. Die weitere Auftrennung der Wasserfraktion erfolgt an Permutit A, einem Anionenaustauscher, wobei zur Elution Wasser beziehungsweise eine mit Kohlensäure gesättigte Lösung eingesetzt wird. Die Purine und Pyrimidine finden sich im Kohlensäureeluat, wohingegen die Wasserfraktion Zucker und andere Neutralstoffe enthält. Eine weitere Isolierung in einzelne Bestandteile findet nicht statt.

Die Anmelderin der vorliegenden Erfindung trennt jährlich große Mengen Zuckerrübenmelasse chromatographisch auf und befasst sich schon seit längerem mit der Möglichkeit, Melasse als Quelle für Uridin einzusetzen. Eine allgemeine Beschreibung eines von der Anmelderin hierfür eingesetzten Verfahrens gibt F. Irtel in seiner Doktorarbeit "Neue Analytik für industrielle Aufarbeitungsprozesse" Universität Hannover, 2001, auf Seiten 89 bis 79. Das Thema der Doktorarbeit betrifft grundsätzlich die Entwicklung von Biosensoren, die eine hohe Selektivität für bestimmte Bestandteile der Melasse, unter anderem Uridin, aufweisen mit der Zielsetzung, diese Biosensoren für die Bestimmung und Überwachung der erforderlichen Trennzeiten der diese Bestandteile enthaltenen Fraktionen bei chromatographischen Auftrennungen einzusetzen. In diesem Zusammenhang wird ohne Angabe näherer Einzelheiten kurz eine chromatographische Aufarbeitung von Melasse zur Gewinnung von Uridin skizziert.

Hierzu wird die Melasse, wie bei der Melasseentzuckerung üblich zur Abtrennung der Saccharose, zunächst einer lonenausschluss-Chromatographie unterzogen, wobei als Trennmittel ein für die Melasseentzuckerung grundsätzlich bekanntes monodisperses sulfoniertes Polystyrol-Divinylbenzol-Harz und als Elutionsmittel Wasser eingesetzt werden. Die unterschiedlichen Fraktionen der Ionenausschluss-Chromatographie werden anschließend über einen Anionenaustauscher geleitet, wobei angemerkt ist, dass in Abhängigkeit des Elutionsmittels eine mit Uridin angereicherte Fraktion erhalten wird. Weitere Angaben zu den Elutionsmitteln, insbesondere konkrete Beispiele finden sich nicht. In einer nächsten Stufe erfolgt dann eine Chromatographie über ein sogenanntes Tauscher-Rack zur Abtrennung von Verunreinigungen wie Asche und der für die charakteristische braune Färbung der Melasse verantwortlichen Bestandteile. Nach Aufkonzentration der Uridinfraktion durch Eindampfen und weiterer Reinigung durch Entfärbung und Filtration muss für das dort beschriebene Verfahren vor der Kristallisation eine weitere Anreicherung des Uridins durch eine lonenaustausch-Chromatographie erfolgen.

Weder werden Angaben zur Abtrennung von Uracil von Uridin gemacht, noch findet sich ein Hinweis auf die tatsächliche erzielte Anreicherung an Uridin. In diesem Zusammenhang wird pauschal lediglich darauf hingewiesen, dass zum Zeitpunkt der Anionenaustausch-Chromatographie der Uridingehalt 1-10 g/L beträgt.

Für die Verwendung des gewonnenen Uridins insbesondere im Bereich der Pharmazie ist jedoch eine hohe Reinheit unabdingbare Voraussetzung. Untersuchungen zur Uridinkristallisation haben gezeigt, dass bereits bei einem Uracilgehalt von nur 2 g/100g Uridin das erhaltene Kristall Uracil in nicht tolerierbarer Konzentration enthält.
Neben einer hohen Reinheit sollte Uridin kostengünstig in einem wirtschaftlichen Prozess in hoher Ausbeute erhalten werden können. Für eine ökonomisch sinnvolle Prozessführung sollte hierbei Uridin bereits in frühen Verfahrensschritten hoch angereichert werden können, so dass der Verarbeitungsaufwand reduziert werden kann.

Erfindungsgemäß wird ein Verfahren zur Verfügung gestellt, mit dem Uridin aus Melasse oder einer uridinhaltigen Melassefraktion wirtschaftlich in hoher Ausbeute und in hoher Reinheit erhalten werden kann. Insbesondere ermöglicht das erfindungsgemäße Verfahren eine Abtrennung der entsprechenden Nukleobase Uracil von der Uridinfraktion.
Weiter sollte ein wirtschaftliches Verfahren zur Uridingewinnung die Gewinnung mit einfachen Trennschritten ermöglichen, ohne dass aufwendige gezielte Synthesen oder Stofftrennungen notwendig sind.

Erfindungsgemäß wird diese Aufgabe durch ein Verfahren gelöst, gemäß dem Melasse oder eine uridinhaltige Melassefraktion in einer ersten Stufe über einen ersten stark basischen Anionenaustauscher geleitet wird, wobei der Anionenaustauscher Uridin bindet, eine uridinhaltige Fraktion von dem Austauscher eluiert wird, und das erhaltene uridinhaltige Eluat einer Ionenausschluss-Chromatographie an einem stark sauren Kationenaustauscher unterzogen wird.

Gemäß einem weiteren Aspekt betrifft die Erfindung ein Verfahren zur Abtrennung der Nukleoside von den entsprechenden Nukleobasen mittels Ionenausschluss-Chromatographie.

Für die vorliegende Erfindung erfolgt die Einteilung in stark/schwach, saure/basische Kationen-/Anionenaustauscher nach den hierfür in der Fachwelt allgemeinen anerkannten Kriterien.

Für das erfindungsgemäße Verfahren zur Gewinnung von Uridin wird Melasse als Ausgangssubstanz eingesetzt, ein Rohstoff, in dem Uridin in freier Form vorliegt.
Für das erfindungsgemäße Verfahren kann prinzipiell jede uridinhaltige Melassefraktion verwendet werden. Ist der Salzgehalt der Melassefraktion, die als Ausgangsmaterial verwendet wird, zu hoch, sollte der Salzgehalt der Melassefraktion vor Behandlung am Anionenaustauscher reduziert werden, da sehr hohe Salzgehalte eine erhöhte Menge an Anionenaustauschermaterial notwendig machen und somit die Wirtschaftlichkeit verringert wird. Die Salzabreicherung kann mit beliebigen hierfür bekannten Mitteln erfolgen.
Vorzugsweise wird eine uridinhaltige, gegebenenfalls salzreduzierte Melassefraktion eingesetzt, wie sie bei der grundsätzlich bekannten Melasseentzuckerung mittels lonenausschluss-Chromatographie an stark sauren Kationenaustauschern anfällt.
Es hat sich gezeigt, dass sich hierbei Uridin in einem gewissen Gehalt von etwa 200 mg/100 g Trockensubstanz in der Saccharosefraktion anreichert, so dass für das erfindungsgemäße Verfahren bevorzugt die bei der üblichen Melasseentzuckerung erhaltene Saccharosefraktion eingesetzt wird. Optional kann damit das erfindungsgemäße Verfahren als vorgeschalteten Schritt eine lonenausschluss-Chromatographie, wie sie im Rahmen der Melasseentzuckerung durchgeführt wird, für eine ersten Anreicherung des Uridins in einer geeigneten Fraktion umfassen.

Neben Uridin finden sich in der Melasse beziehungsweise der uridinhaltigen Melassefraktion eine Reihe weiterer Komponenten, zu denen insbesondere Uracil zählt.

Zur weiteren Anreicherung an Uridin und Abtrennung der weiteren Bestandteile wird das gewünschte uridinhaltige Ausgangsmaterial einem lonenaustauschprozess an einem ersten Anionenaustauscher unterzogen. Aufgrund seines schwachen Säurecharakters, der sich aus der Dissoziation des Imids ergibt, bindet Uridin an den Anionenaustauscher.

Aufgrund ihrer hohen Stabilität gegen organisches Fouling sind geeignete Anionenaustauscher zum Beispiel makroporöse Polyacrylate, die als funktionelle Gruppe quateres Amin enthalten. Beispiele hierfür sind Anionenaustauscher, wie sie unter der Bezeichnung Purolite A 860 S oder Amberlite IRA 958 vertrieben werden. Der Anionenaustauscher liegt hierbei in der OH⁻-Form vor.

Zur Elution kann prinzipiell ein beliebiges Elutionsmittel verwendet werden, das Uridin vom Tauscher löst. Geeignete Elutionsmittel sind Basen und Säuren. Es hat sich jedoch gezeigt, dass der Grad der Anreicherung des Uridins sowie dessen Reinheit mit der Art des gewählten Elutionsmittels variiert.

Erfolgt die Elution mit einer Base, wie 5 %iger Natronlauge, findet sich im erhaltenen Eluat Uridin gemeinsam mit allen anderen adsorbierten Stoffen einschließlich der Farbstoffe. Der Gehalt an Uridin beträgt hierbei 25 g/100 g Trockensubstanz.

Die Elution mit Säure führt demgegenüber zu einer weitgehenden Trennung des Uridins von den anderen, aus der Zuckerlösung separierten Bestandteilen. Überraschenderweise hat sich gezeigt, dass bei Verwendung einer Säure, insbesondere einer verdünnten Säure, der Uridingehalt des Eluats gegenüber dem basischen Eluat erheblich gesteigert werden kann und ein Eluat mit einem Uridingehalt von etwa 70 g/100 g Trockensubstanz erhalten werden kann.
Erfindungsgemäß bedeutet hierbei "verdünnte Säure" eine Säure mit einem Gehalt von 0,2 bis 3 Äquivalenten/Liter und insbesondere von 0,5 bis 1 Äquivalent/Liter.

Beispiele für geeignete Säuren sind Schwefelsäure, Salzsäure und Essigsäure.

In dem Eluat der verdünnten Säure findet sich das Uridin hierbei zusammen mit Uracil und gegenüber der basischen Elution verringerten Mengen an weiteren Bestandteilen wie Melassefarben, Asche bildenden Salzen, Aminosäuren und Saccharose.

Wie jedoch bereits vorstehend ausgeführt, führt bereits ein Uracilgehalt von nur 2 g/100 g Uridin bei Kristallisation von Uridin zu Kristallen, die Uracil in einer für die gewünschten Anwendungszwecke nicht tolierbaren Konzentration enthält.

Erfindungsgemäß wurde nunmehr gefunden, dass Uracil von Uridin abgetrennt beziehungsweise der Gehalt an Uracil sich maßgeblich reduzieren läßt, indem das erhaltene Eluat mit Uridin und Uracil einer lonenausschluss-Chromatographie unterzogen wird. Neben der Abtrennung des Uracils beziehungsweise Reduzierung des Uracilgehalts lassen sich hierbei auch gegebenenfalls vorhandene weitere Bestandteile weitestgehend abtrennen.

Erfindungsgemäß erfolgt die lonenausschluss-Chromatographie zur Uracilabtrennung an einem stark sauren Kationenaustauscher.
Besonders gute Ergebnisse lassen sich mit Alkalimetallionen abgesättigten, gelförmigen Kationenaustauscher auf Basis sulfonierten Polystyrols mit einem Wassergehalt von 40 bis 65 %, insbesondere von 45 bis 60 %, sowie einer mittleren Korngröße von 200 bis 450 µm und einem Uniformitätskoeffizienten von < 1,6, insbesondere < 1,2, erhalten.
Die Elution erfolgt zweckmäßigerweise mit Wasser, da das gesamte Verfahren vorzugsweise auf wässrige Lösungen abgestimmt ist, wie sie üblicherweise auch bei der Melasseentzuckerung anfallen.

Es hat sich gezeigt, dass bei einem pH-Wert-Bereich von 5 bis 8 sehr gute Ergebnisse erhalten werden können, sodass dieser Bereich für die Durchführung der lonenausschluss-Chromatographie zur Abtrennung von Uracil von Uridin bevorzugt ist. Aufgrund der besonders einfachen Handhabung wird besonders bevorzugt im neutralen Bereich gearbeitet.

Aus dem erhaltenen Eluat läßt sich Uridin in einer Ausbeute von 50 % und mehr in einer Reinheit von mindestens 96 g/100 g Trockensubstanz kristallisieren. Selbstverständlich kann die Isolierung des Uridins aus dem Eluat auch auf jede andere geeignete Weise durchgeführt werden.

Bei Bedarf kann das erfindungsgemäße Verfahren mit weiteren Trennverfahren kombiniert werden, bei denen in den jeweiligen Fraktionen enthaltene weitere Melassebestandteile wie zum Beispiel die Melassefarben, aschebildende Salze, Aminosäuren oder Oligosaccharide ganz oder teilweise abgetrennt werden, um so das erfindungsgemäß eingesetzte System mit erstem Anionenaustauscher und lonenausschluss-Chromatographie zu entlasten.
Vorzugsweise werden hierfür ebenfalls lonenaustauschverfahren eingesetzt.
So kann die für den ersten Anionenaustauscher vorgesehene uridinhaltige Fraktion vor Aufgabe auf den ersten Anionenaustauscher auf einen Kationenaustauscher gegeben werden. Als Trennmittel für den Kationenaustauscher können sulfonierte Polystyrole verwendet werden. Diese sollten in der H⁺-Form vorliegen, um so eine gleichzeitige Entsalzung und Entfärbung der Fraktion zu erzielen. Falls gewünscht, kann die Fraktion auch über eine Folge von derartigen Kationen- und den erfindungsgemäßen Anionenaustauschern gegeben werden.

Auch hat es sich bewährt, das uridinhaltige Eluat aus dem ersten Anionenaustauscher vor Auftrennung mit der lonenausschluss-Chromatographie einer Behandlung an einem stark sauren Kationenaustauscher, vorzugsweise gefolgt von einem schwach basischem Anionenaustauscher, zu unterziehen. Auf diese Weise wird eine Vollentsalzung des Eluats und damit eine Entlastung der lonenausschluss-Chromatographie erhalten. Alternativ oder bei Bedarf auch zusätzlich kann die Behandlung mit der Kombination aus stark sauren Kationenaustauscher und/oder schwach basischem Anionenaustauscher der lonenausschluss-Chromatographie auch nachgeschaltet sein.

Als stark sauerer Kationenaustauscher können sulfonierte Polystyrole in H⁺-Form verwendet werden. Beispiele hierfür sind Kationenaustauscher, wie sie unter der Bezeichnung Amberlite IRA 120 und Levati S 100 vertrieben werden. Als schwach basische Anionenaustauscher können Polymere eingesetzt werden, die überwiegend mit tertiären Aminen funktionalisiert sind und in freier Baseform vorliegen. Bevorzugte schwach basische Anionenaustauscher sind makroporöse Polystyrole, die ausschließlich mit tertiärem Amin funktionalisiert sind.

Vorzugsweise wird das Eluat des ersten Anionenaustauschers zur Abtrennung von Fremdsalzen und Farbstoffen so lange über die vorstehend genannte Kombination aus stark sauren Kationenaustauscher gefolgt von einem schwach basischem Anionenaustauscher geleitet, bis einer der Tauscher nicht mehr in der Lage ist, die abzutrennenden Ionen zu binden.

Die durch die erfindungsgemäße lonenausschluss-Chromatographie bewirkte Abtrennung von Uridin von seiner Nukleobase Uracil ist nicht auf das System Uridin/Uracil beschränkt, sondern eignet sich prinzipiell zur Abtrennung von Nukleosiden von Nukleobasen.
So können zum Beispiel mit den vorliegend beschriebenen Maßnahmen zur Durchführung einer lonenanschluss-Chromatographie generell Nukleoside von den entsprechenden Nukleobasen abgetrennt werden.
Die vorliegende Erfindung umfasst damit auch ein Verfahren zur Auftrennung von Nukleosiden und Nukleobasen mittels Ionenausschluss-Chromatographie.

Die Isolierung des Uridins aus der uridinhaltigen Fraktion der erfindungsgemäßen lonenausschluss-Chromatographie kann grundsätzlich auf beliebige hierfür geeignete Art und Weise erfolgen.

Vorzugsweise erfolgt die Isolierung mittels Kristallisation. Als besonders geeignet hat sich die Durchführung der Kristallisation als Kühlüngskristallisation erwiesen. Hierfür wird eine Lösung eingesetzt, die bei über 40 °C übersättigt sein sollte.
Vorzugsweise zeigt die Lösung bei 30 °C und insbesondere bevorzugt bei weniger als 20 °C Übersättigung. Die maximale Kühlrate sollte hierbei 5 °C, vorzugsweise 2 °C und insbesondere 0,5 °C nicht überschreiten.

Falls gewünscht, kann das erhaltene kristalline Produkt einer oder mehreren Umkristallisationen zur weiteren Erhöhung der Reinheit des Uridins unterzogen werden.

Das erfindungsgemäß erhaltene hochreine Uridin kann unmittelbar oder, falls gewünscht, nach Derivatisierung allein oder in Kombination mit ein oder mehreren weiteren Wirkstoffen eingesetzt werden.
Bekannte Einsatzgebiete umfassen pharmazeutische Anwendungen und die Verwendung als Nahrungsmitteladditiv oder -zutat, wie sie aus technologischen Gründen Lebensmitteln zugesetzt werden, sowie die Verwendung aus ernährungsphysiologischen Gründen in Nahrungsergänzungsmitteln oder Functional Food.
Darüber hinaus ist Uridin aufgrund seiner regulatorischen Einflüsse auf biologische Systeme nicht auf die vorgehend genannten Anwendungen beschränkt, sondern eignet sich hervorragend für kosmetische Zwecke als Zusatzstoff für Tierfutter und Heimtiernahrung oder als Lebensmittelzusatz.
Wie bei den grundsätzlich bekannten Anwendungen in der Pharmazie beziehungsweise als Nahrungsmitteladditiv kann das Uridin hierbei als Uridinderivat sowie ggf. in Kombination mit ein oder mehreren weiteren Wirkstoffen vorliegen.
Gemäß einem weiteren Aspekt umfasst die Erfindung damit die Verwendung von Uridin und Uridinderivaten in Kosmetika und als Zutat oder Zusatzstoff für Tierfutter und Heimtiernahrung.

Die nachstehenden Beispiele dienen der Erläuterung der vorliegenden Erfindung.

### Beispiel 1

105 l einer aus der chromatographischen Melasseentzuckerung stammenden Saccharosefraktion mit 0,04 % Uridin sowie 16,5 % Trockensubstanz (TS) wurden über 5,7 l des stark basischen in OH⁻-Form befindlichen Anionenaustauscher Purolite A 860 geleitet.
Der Tauscher wird mit 6 l voll entsalztem Wasser gewaschen.
Zur Elution des Uridins wurden 2,5 l einer 4 %igen Schwefelsäure auf den Tauscher gegeben.

Das erhaltene Eluat wurde mit Hilfe eines Probenahmesystems in 25 gleich große Fraktionen aufgeteilt, wobei alle Fraktionen mit einem TS-Gehalt von < 1 % als Produktfraktion vereinigt wurden. Die erhaltene Produktfraktion umfaßte 4 l. Diese Lösung wies einen TS-Gehalt von 2,5 % sowie einen Uridingehalt von 1,72 % auf. Damit konnte die Uridinreinheit von 0,24 auf 69 % erhöht werden.

### Beispiel 2

336,1 l einer analog Beispiel 1 erhaltenen Lösung mit 1,17 % Uridin und 2 % TS wurden über 15 l des in H⁺-Form befindlichen Kationenaustauschers Levatit S 100, gefolgt von 16 l eines in der freien Baseform befindlichen schwach basischen Anionenaustauschers Amberlite IRA 93 geleitet.
Der Durchlauf wurde vereinigt und auf 37,4 % TS konzentriert. Das Konzentrat enthielt Uridin einer Reinheit von 71 % bei einer Ausbeute von 97 %.

Das Konzentrat wurde auf 80 °C erhitzt und mit 10 g Aktivkohle Norit CA 1 pro 100 g TS zur Entfärbung versetzt. Anschließend wurde die Kohle bei 80 °C über ein Tuchfilter abgetrennt.

### Beispiel 3

1 kg des im Beispiel 2 erhaltenen Filtrats wurde bei 80 °C einer lonenausschluss-Chromatographie an 16 des stark sauren, in Na⁺-Form befindlichen Kationenaustauschers Levatit MDS 1368 unterzogen.
Die zur Chromatographie eingesetzte Trennsäule wies einen Innendurchmesser von 8 cm auf. Als Eluiermittel wurde demineralisiertes Wasser mit 80 °C bei einer Flussrate von 8 l/h verwendet.

Der erhaltene Auslauf der lonenausschluss-Chromatographie wurde mit Hilfe eines Probenahmesystems fraktioniert. Die erhaltenen Produktfraktionen wurden ab Anstieg des Trockensubstanzsgehaltes über 1 % bis zum Abfall des Trockensubstanzgehaltes unter 7,5 % geschnitten. Die Trennung mittels lonenausschlusschromatographie wurde fünfmal wiederholt. Die Produktfraktionen wurden vereinigt. Der Uridingehalt der vereinigten Produktfraktion betrugt 85 g/100 g TS. Die Uridinausbeute betrug 70 %.

Die vereinigte Produktfraktion wurde bei 70 °C unter Vakuum auf einen Trockensubstanzgehalt von 62 g/100 g Lösung konzentriert und anschließend zur Kristallisation auf 25 °C abgekühlt.
Die erhaltenen Kristalle wurden unter Vakuum auf einem Tuchfilter abgetrennt und analysiert. Die Kristalle enthielten 49 % der enthaltenen Uridinmenge bei einer Reinheit von 99 g/100 g TS.

Die erhaltene Mutterlösung wurde wiederum bei 70 °C auf 76 % TS konzentriert. Nach Abkühlung auf 40 °C und Kristallisierung erfolgte die Kristallabtrennung wiederum über einen Tuchfilter. In dieser Nachkristallisation wurden 43 % des in der Mutterlösung der ersten Kristallisation verbliebenen Uridins mit einer Reinheit von 95 g/100 g TS gewonnen.

### Vergleichsbeispiel

Eine uridinhaltige Lösung mit einem Gehalt 83,6 g Uridin/100 g Lösung wurde analog den Beispielen 1 und 2 hergestellt. Die erhaltene Lösung wurde unmittelbar der Kristallisation unterzogen. Hierzu wurde die Lösung unter Vakuum bei 70 °C zur direkten Kristallisation auf einen großen TS-Gehalt von 81,8 % konzentriert. Zur Kristallisation wurden dann 96 g der Lösung langsam auf 30 °C abgekühlt. Die erhaltenen Kristalle wurden mit einer Siebzentrifuge abgetrennt und analysiert. Die Kristalle enthielten 46 % der eingesetzten Uridinmenge mit einer Reinheit von 86 g/100 g.

## Patentansprüche

1. Verfahren zur Gewinnung von Uridin aus Melasse oder einer uridinhaltigen Melassefraktion als Ausgangsmaterial,
**dadurch gekennzeichnet,**
**dass** das Ausgangsmaterial über einen ersten stark basischen Anionenaustauscher geleitet wird, das an den ersten Anionenaustauscher gebundene Uridin eluiert wird, und das erhaltene uridinhaltige Eluat einer lonenausschluss-Chromatographie an einem stark sauren Kationenaustauscher als Trennmittel unterzogen wird,
und gegebenenfalls der Salzgehalt der Melasse oder uridinhaltigen Melassefraktion vor Aufgabe auf den ersten Anionenaustauscher reduziert wird.

2. Verfahren nach Anspruch 1,
**dadurch gekennzeichnet,**
**dass** die uridinhaltige Melassefraktion eine Saccharosefraktion ist, die erhalten wird, indem Melasse einer lonenausschluss-Chromatographie zur Melasseentzuckerung unterzogen wird.

3. Verfahren nach Anspruch 1 oder 2,
**dadurch gekennzeichnet,**
**dass** der erste Anionenaustauscher ein makroporöses Polyacrylat mit quaterem Amin als funktioneller Gruppe ist.

4. Verfahren nach einem der vorhergehenden Ansprüche,
**dadurch gekennzeichnet,**
**dass** zur Elution des Uridins von dem ersten Anionenaustauscher ein saures Elutionsmittel verwendet wird.

5. Verfahren nach Anspruch 4,
**dadurch gekennzeichnet,**
**dass** als saures Elutionsmittel eine Mineralsäure verwendet wird.

6. Verfahren nach Anspruch 5,
**dadurch gekennzeichnet,**
**dass** der Säuregehalt der Mineralsäure in einem Bereich von 0,2 bis 3 Äquivalenten/Liter liegt.

7. Verfahren nach einem der vorhergehenden Ansprüche,
**dadurch gekennzeichnet,**
**dass** das Trennmittel ein gelförmiger Kationenaustauscher auf Basis von sulfonierten Polystyrol mit einem Wassergehalt von 40 bis 65 % ist, der mit Alkalimetallionen abgesättigt ist.

8. Verfahren nach einem der vorhergehenden Ansprüche,
**dadurch gekennzeichnet,**
**dass** das Trennmittel eine mittlere Korngröße in einem Bereich von 200 bis 450 µm mit einem Uniformitätskoeffizienten von < 1,6 aufweist.

9. Verfahren nach einem der vorhergehenden Ansprüche,
**dadurch gekennzeichnet,**
**dass** durch die lonenanschluss-Chromatographie Uracil von der Uridinfraktion abgetrennt wird.

10. Verfahren nach einem der vorhergehenden Ansprüche
**dadurch gekennzeichnet,**
**dass** die Isolierung des Uridins aus der erhaltenen uridinhaltigen Fraktion mittels Kühlungskristallisation erfolgt.

11. Verfahren nach einem der vorhergehenden Ansprüche,
**dadurch gekennzeichnet,**
**dass** das Verfahren weitere Trennverfahren zur Entfernung von Fremdbestandteilen zur Unterstützung umfasst.

12. Verfahren nach Anspruch 11,
**dadurch gekennzeichnet,**
**dass** das uridinhaltige Eluat des ersten Anionenaustauschers zur Abtrennung von Fremdsalzen und Farbstoffen über eine Kombination aus einem stark sauren Kationenaustauscher gefolgt von einem schwach basischen Anionenaustauscher geleitet wird.

13. Verfahren nach Anspruch 11 oder 12,
**dadurch gekennzeichnet,**
**dass** das uridinhaltige Ausgangsmaterial vor Aufgabe auf den ersten Anionenaustauscher auf einen Kationenaustauscher gegeben wird.

14. Verfahren nach Anspruch 12 oder 13,
**dadurch gekennzeichnet,**
**dass** die Folge von Kationen- und Anionenaustauschern zweifach oder mehrfach durchlaufen wird.

15. Verfahren zur Abtrennung von Nukleobasen von Nukleosiden,
**dadurch gekennzeichnet,**
**dass** die Abtrennung mittels lonenausschluss-Chromatographie an einem stark sauren Kationenaustauscher erfolgt.

16. Verwendung von Uridin allein oder in Kombination mit einem oder weiteren Wirkstoffen in kosmetischen Zubereitungen als Zusatz zu Tierfutter und Heimtiernahrung oder als Lebensmittelzusatz.
